# EUROPEAN PATENT APPLICATION

(11) **EP 2 289 903 A2**
(43) Date of publication of application: **02.03.2011**
(21) Application number: 10167113.9
(22) Date of filing: 24.06.2010
(51) Int. Cl.: C07H 1/08

(54) **Method and device for the preparation of the sodium salt of deoxyribonucleic acid and related product**

(30) Priority: 24.06.2009 IT BS20090116
(71) Applicant: Merighi, Cesare, 25123 Brescia (IT)
(72) Inventor: Merighi, Cesare, 25123 Brescia (IT)
(74) Representative: Pulieri, Gianluca Antonio

(57) **Abstract**

The present invention relates to a method for the preparation of the sodium salt of deoxyribonucleic acid (DNA) comprising the steps of supplying a de-fatted phase containing DNA molecules, supplying at least one primary solution, containing sodium chloride, suitable for solubilising the de-fatted phase or for suspending it, and feeding the de-fatted phase and the primary solution to a primary mixing reactor so as to obtain a de-fatted mixture.

The method further comprises a step of cooling the de-fatted mixture to a predefined cooling temperature, which comprises a step of taking a portion of the de-fatted mixture from the primary reactor, and a step of reintroducing such portion into it.

## Description

. The present invention relates to a method and a device for the preparation of the sodium salt of deoxyribonucleic acid, and to the related product.

. A variety of methods for the preparation of the sodium salt of deoxyribonucleic acid (DNA) are known.

. Such methods usually envisage a step of supplying a de-fatted phase containing molecules of DNA, a step of mixing the de-fatted phase with a solution containing sodium chloride, suitable for solubilising the de-fatted phase, and a step of cooling such mixture to a pre-set temperature.

. However, the known methods and corresponding devices suffer a plurality of drawbacks.

. In particular, such methods often prove ineffective in controlling the heat during mixing.

. In fact, the mixture needs to be cooled as rapidly as possible, and the subsequent processing steps have to be performed within narrow temperature ranges, without fluctuations of such parameter.

. The present invention therefore sets out to solve the problems of the prior art and, in particular, those mentioned above.

. These drawbacks are solved by a method according to claim 1 and by means of a device according to claim 14. The dependent claims describe preferred embodiments.

. The present invention will now be described in detail with the help of the attached drawings, wherein:

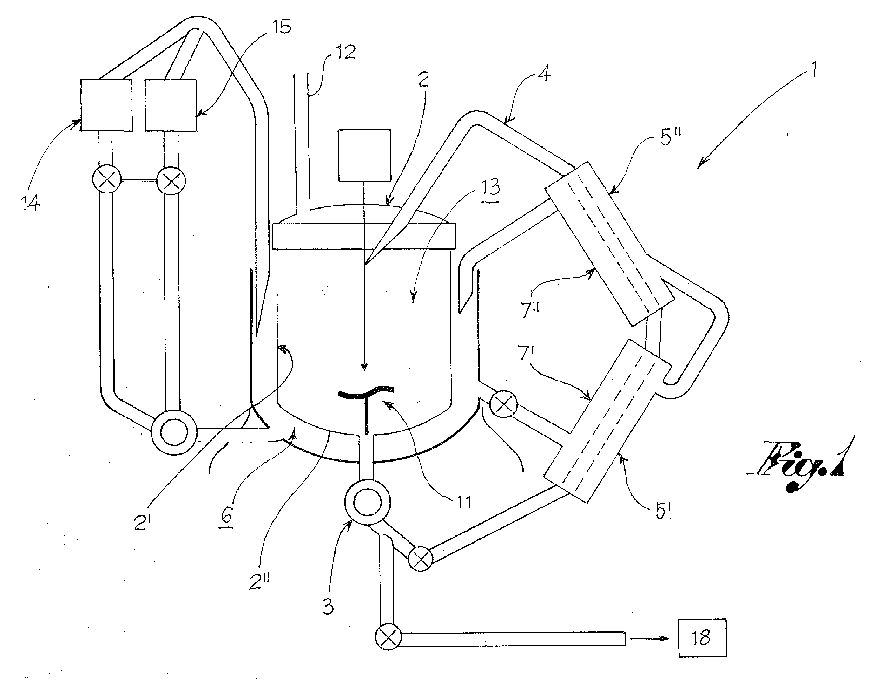

. - Figure 1 shows a schematic view of a first piece of equipment used in the device of the present invention, according to a possible embodiment;

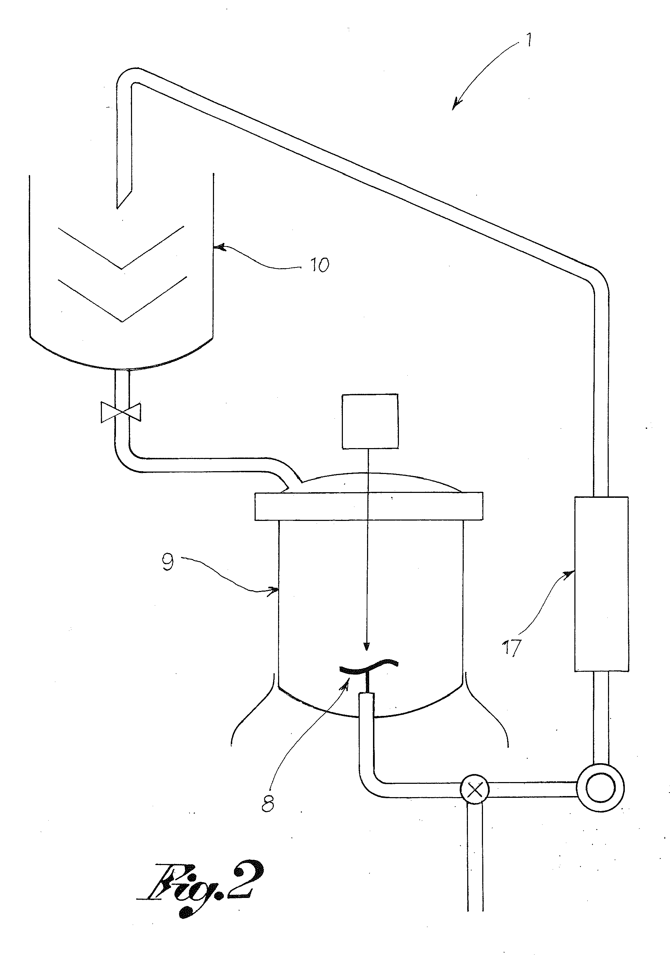

. - Figure 2 shows a schematic view of a further piece of equipment used in the device of the present invention, according to a variation.

. With reference to the aforementioned drawings, reference numeral 1 globally indicates a device for the preparation of the sodium salt of deoxyribonucleic acid (DNA).

. The device 1 comprises a primary mixing reactor 2, suitable for being fed with a de-fatted phase containing molecules of DNA and with at least one primary solution containing sodium chloride, to obtain a de-fatted mixture.

. In other words, in the primary mixing reactor 2 the de-fatted phase is mixed with the primary solution to obtain a de-fatted mixture.

. Preferably, the primary reactor 2 comprises primary homogenisation means, such as a mechanical 11 or magnetic stirrer, to promote homogenisation of the de-fatted mixture.

. According to the embodiment shown in figure 1, the device 1 further comprises feeding means of the de-fatted phase and/or of the primary solution to the primary mixing reactor 2, for example at least one feeding duct 12.

. The feeding means are therefore used to fill the primary reactor 2.

. The device 1 further comprises heat exchange means, operatively connected to the primary reactor 2 to regulate its temperature.

. According to a preferred embodiment, the primary reactor 2 delimits a heat exchange cavity 6 suitable for the passage of a heat exchange liquid that can be heated or cooled. According to such variation, the heat exchange means comprise the heat exchange cavity 6.

. In other words, the wall of the primary reactor 2 has an inner surface 2', facing the containment compartment 13 of the de-fatted mixture, in thermal connection with such mixture, and an outer surface 2", facing outwardly from the containment compartment 13 in thermal connection with the heat exchange liquid.

. This way, advantageously, the heat exchange liquid influences the temperature of the de-fatted mixture without reciprocal contamination of the two fluids.

. Preferably, the heat exchange means comprise at least one heat exchanger 7', 7" through which the de-fatted mixture and a heat exchange liquid pass, in reciprocal thermal contact.

. Even more preferably, the heat exchanger 7', 7" is a separate piece of equipment from the primary reactor 2.

. According to the embodiment shown in figure 1, the heat exchange liquid circulating in the heat exchanger 7', 7" comes from the heat exchange cavity 6.

. According to a preferred variation, the device 1 further comprises heating means 14 and/or cooling means 15, suitable for heating or cooling the heat exchange liquid, and therefore the de-fatted mixture, during the different method steps.

. As a result, according to various embodiments, the heating 14 and/or cooling 15 means feed in series, or in parallel, the heat exchange cavity 6 and the heat exchanger 7', 7".

. The device 1 further comprises circulation means 3, 4 suitable for taking a portion of the de-fatted mixture from the primary reactor 2, and for reintroducing such portion inside it.

. Innovatively, the device of this invention allows improved heat control of the de-fatted mixture in that, by reducing the quantity of such mixture inside the primary reactor, the heat exchange means need to work less to cool/heat it.

. Preferably, the circulation means 3, 4 take the portion of de-fatted mixture from the primary reactor 2 conveying it outside the primary reactor 2.

. This way, advantageously, the thermal equilibrium between the heat exchange liquid and the de-fatted mixture is reached faster, thanks to the heat dispersion which the de-fatted mixture undergoes during its path externally from the primary reactor 2.

. Preferably, the circulation means comprise at least one peristaltic pump 3.

. According to one variation, the circulation means further comprise at least one circulation duct 4, in fluidic connection with the containment compartment 13, suitable for taking, e.g. from the bottom of the primary reactor 2, the portion of de-fatted mixture and for reintroducing it.

. Preferably, the device 1 further comprises crushing means 5', 5" operatively connected to the primary reactor 2 so as to crush the molecules of DNA contained in the de-fatted mixture.

. As a result, the crushing means 5', 5" are suitable for reducing the dimensions of the DNA molecules contained in the de-fatted mixture to a desired molecular size.

. Preferably, the desired molecular size is within the range 250-400 U (unified atomic mass unit) and, even more preferably, in the range 270-350 U.

. According to advantageous variations of the invention, the desired molecular size is approximately 250-270 U, 271-290 U, 291-310 U, 311-330 U, 331-350 U, 351-370 U, 371-390 U and/or 391-400 U.

. Even more preferably, the crushing means 5', 5" comprise ultrasound means, advantageously piezoelectric ultrasound generators.

. According to a preferred embodiment, the crushing means 5', 5" and the heat exchanger 7', 7" are integrated in a single assembly.

. This way, advantageously, the heat intrinsically generated by the crushing means 5', 5" is dissipated by the heat exchanger 7', 7", without such heat jeopardising the condition of the de-fatted mixture.

. Preferably, the device 1 further comprises analysis means to determine the crushing degree of the DNA molecules.

. Even more preferably, the analysis means comprise a viscometer, advantageously of the torsion type.

. In fact, a specific viscosity of the de-fatted mixture is associated with a specific molecular size of the DNA molecules.

. This way, advantageously, it is possible to analyse, e.g. continuously, the de-fatted mixture viscosity, and stop crushing the DNA molecules when they have reached the desired size.

. Preferably, the device 1 further comprises shredding means 8, suitable for reducing the dimensions of an organic raw material, e.g. sturgeon and/or salmon gonads.

. E.g. the shredding means comprise a mincer, a whisker, a rotating knife, a mixer or similar.

. As a result, the organic raw material is fed whole or in a coarse state to a secondary mixing reactor 9, and is shredded by the shredding means 8, preferably positioned inside such reactor 9.

. In fact, according to an advantageous embodiment, the device 1 comprises a secondary mixing reactor 9, suitable for being fed with the organic raw material, preferably shredded, and with a secondary solution comprising sodium chloride and/or citrate, to obtain an intermediate mixture.

. In other words, the secondary mixing reactor 9 is suitable for receiving the organic raw material, shredded or to be shredded, and the secondary solution, so as to mix such components.

. Preferably, the step of mixing happens at least partially at the same time as step of shredding.

. This way, the intermediate mixture which goes through subsequent processing is obtained.

. Preferably, the device 1 comprises separator means 10, suitable for separating the intermediate mixture into the de-fatted phase, containing DNA molecules, and into a phase containing the fat substances.

. Advantageously, the presence of separator means 10 allows to divide the intermediate mixture into a useful phase, the de-fatted phase, which continues on for subsequent processing, and a phase containing the fat substances which is taken away.

. Preferably, the separator means 10 comprise centrifuge means.

. With reference to the schematisation of figure 2, the separator means 10 comprise a centrifuge wherein the intermediate mixture is received.

. During centrifugation, this mixture is separated into the de-fatted phase, which sediments, and the phase containing the fat substances which comes out of the centrifuge, e.g. through a spillway (not shown), by effect of the centripetal force.

. Preferably, the device 1 comprises secondary homogenisation means 17, suitable for homogenising the intermediate mixture.

. Even more preferably, the secondary homogenisation means 17 are positioned downstream, in relation to the intermediate mixture flow, of the secondary mixing reactor 9.

. E.g. the secondary homogenisation means 17 comprise at least a device known by the brand name Ultra-Turrax®.

. Preferably, the secondary homogenisation means 17 are suitable for feeding the separator means 10 so that, after the separation of the fat substances, the intermediate mixture is reintroduced into the secondary mixing reactor 9, and mixed with further secondary solution.

. Even more preferably, the mixing step in the secondary mixing reactor 9, the homogenisation step using the secondary homogenisation means 17 and the separation step using the separator means 10 are repeated until a desired purity of the de-fatted phase is achieved.

. Advantageously, such operations are repeated in a substantially automated manner making the intervention of on-site operators minimal or unnecessary.

. Furthermore, the three aforementioned steps used in succession allow excellent yields, especially in terms of separation of the fat substances, in extremely short times compared to the manual method traditionally used.

. In fact, the prior art envisaged sequential processing of small amounts of intermediate mixture, and the repeated performance of substantially handmade methods.

. According to a preferred variation, the device 1 further comprises concentrator means, suitable for concentrating the de-fatted mixture so as to select the DNA molecules having a mass greater than a predefined molecular mass.

. Preferably, the concentrator means comprise filters for ultra-filtration under pressure 18, only schematically shown in figure 1.

. According to advantageous embodiments, the predefined molecular mass is over 40 U, preferably over 50 U, even more preferably over 60 U and, advantageously, over 70 U.

. According to one embodiment variation, the device 1 further comprises crystallisation means, for example from ethanol, suitable for crystallising the DNA molecules having a mass greater than the predefined molecular mass.

. The present invention further relates to a method for the preparation of the sodium salt of deoxyribonucleic acid (DNA).

. Such method comprises a step of supplying the de-fatted phase containing DNA molecules, supplying at least one primary solution, containing sodium chloride, suitable for solubilising the de-fatted phase or for suspending it, and feeding the de-fatted phase and the primary solution into the primary mixing reactor 2 so as to obtain a de-fatted mixture.

. According to a preferred embodiment, the primary solution forms a hypertonic environment.

. Preferably, the step of solubilising the de-fatted phase or suspending it comprises a step of cytolysis, i.e. breaking a cellular wall.

. In fact, the DNA molecules contained in the de-fatted phase are enclosed inside the cellular wall of a cell.

. Consequently, when the cell is placed in a hypertonic environment, the water tends to pass through the cellular wall by osmosis, initially causing an increase in the pressure inside the cell.

. Subsequently, after the pressure exceeds a predefined value, the cell swelling is such as to cause the wall breaking.

. Preferably, the concentration of the sodium chloride in the primary solution is over 4 moles/litre.

. According to advantageous embodiments of the invention, such concentration is between 4 to 15 moles/litre, between 4 to 8 moles/litre, between 4 to 6 moles/litre.

. Even more preferably, the concentration of the sodium chloride in the primary solution is approximately 4-4,25 moles/litre, 4,25-4,5 moles/litre, 4,5-4,75 moles/litre, 4,75-5 moles/litre, 5-5,25 moles/litre, 5,25-5,5 moles/litre, 5,5-5,75 moles/litre, or 5,75-6 moles/litre.

. According to an advantageous embodiment of the method, the step of solubilising the de-fatted phase or of suspending it further comprises a step of binding the sodium contained in the primary solution to the DNA molecules.

. According to a preferred embodiment, the step of supplying the de-fatted phase comprises a step of supplying an organic raw material, supplying at least one secondary solution, comprising sodium chloride and/or citrate, shredding the organic raw material so as to reduce its size in relation to the original dimensions, mixing the organic raw material and the secondary solution to obtain an intermediate mixture, and separating the intermediate mixture into a de-fatted phase containing DNA molecules, and a phase containing the fat substances.

. Preferably, the organic raw material is made of sturgeon and/or salmon gonads.

. Even more preferably, the step of separating the intermediate mixture comprises at least a step of centrifuging.

. Subsequently, the de-fatted mixture is cooled to a predefined cooling temperature.

. Preferably, the predefined cooling temperature is between 0 and 20°C and, preferably, between 1 and 8°C.

. According to advantageous variations of the invention, the predefined cooling temperature is approximately 0-0.5°C, 0.5-1°C, 1-1.5°C, 1.5-2°C, 2-2.5°C, 2.5-3°C, 3-3.5°C, 3.5-4°C and/or 4-4.5°C.

. Furthermore, the step of cooling comprises a step of taking a portion of the de-fatted mixture from the primary reactor 2, and a step of reintroducing such portion into the primary reactor 2.

. Preferably, the step of taking involves conveying the portion of de-fatted mixture externally to the primary reactor 2.

. This way, advantageously, the de-fatted mixture undergoes heat dispersion which increases the cooling efficiency and speed.

. According to one advantageous embodiment, the portion of de-fatted mixture is conveyed into a heat exchanger 7', 7" to accelerate its cooling.

. Preferably, the method of the present invention further comprises a step of crushing the DNA molecules contained in the de-fatted mixture.

. Preferably, the step of crushing comprises a step of exposing the de-fatted mixture to ultrasounds.

. According to a preferred embodiment, the step of crushing comprises a step of exposing the de-fatted mixture to ultrasounds at an intensity ranging from 20 to 50 Hz and, preferably, from 30 to 45 Hz.

. Particularly advantageous embodiments use ultrasound intensities of 28-30 Hz, 30-32 Hz, 32-34 Hz, 34-36 Hz, 36-38 Hz, 38-40 Hz, 42-44 Hz and/or 44-46 Hz.

. Preferably, the steps of cooling and crushing take place at least partially simultaneously.

. This way, advantageously, the heat originating from the ultrasound generators is dissipated by cooling, without such heat jeopardising the condition of the de-fatted mixture.

. In other words, the cooling step cools both the de-fatted mixture and the ultrasound generators using the same heat exchange means.

. Preferably, the step of feeding occurs in the absence of fossil meals and/or sodium dodecyl-sulphate.

. Advantageously, the method of the present invention occurs, within the framework of responsible sacrifice, without the use of fossil meals.

. In fact, despite fossil meals being highly toxic, they are traditionally used to separate the proteins from the intermediate mixture.

. Preferably, the method of the present invention further comprises a step of analysing the de-fatted mixture to determine the crushing degree of the DNA molecules.

. Even more preferably, the step of analysing comprises a step of measuring the viscosity of the de-fatted mixture, for example using a torsion viscometer.

. Advantageously, the step of cooling is preceded by a step of heating the de-fatted mixture for a predefined time.

. In fact, before cooling, the de-fatted mixture must be cooked.

. Advantageously, the step of heating occurs at a temperature ranging from 40°C to 100°C and, preferably, from 50°C to 90°C.

. The particularly preferred temperature ranges are from 50-65°C, 65-80°C and/or 80-95°C.

. Even more advantageously, the predefined time ranges from 1 second to 5 hours and, preferably, from 1 to 3 hours.

. According to preferred method variations, the predefined time ranges from 30 minutes to 1 hour, from 1 hour to 1,5 hours, from 1,5 to 2 hours, from 2 to 2,5 hours and/or from 2,5 to 3 hours.

. Preferably, the method further comprises a step of concentrating the de-fatted mixture, so as to select the DNA molecules having a greater mass than a predefined molecular mass, as illustrated above.

. Preferably, the step of concentrating is performed by ultra-filtration under pressure.

. Advantageously, the absence of fossil meals significantly reduces the risk of blocking of the filters used for ultra-filtration during this step.

. According to a further preferred variation, the method further comprises a step of crystallising the DNA molecules having a greater mass than the predefined molecular mass.

. Preferably, such crystallisation is made from ethanol.

. The present invention further relates to the sodium salt of deoxyribonucleic acid obtained with the method or the device according to any of the embodiments described.

. The present invention relates, lastly, to a method and a device to obtain a de-fatted phase containing DNA molecules.

. Such method comprises the steps of supplying an organic raw material, e.g. sturgeon and/or salmon gonads, supplying at least one secondary solution, comprising sodium chloride and/or citrate, shredding the organic raw material so as to reduce its size from the original dimensions, mixing the organic raw material and the secondary solution to obtain an intermediate mixture, and separating, preferably by centrifuging, the intermediate mixture into the de-fatted phase, containing DNA molecules, and a phase containing the fat substances.

. Such device comprises a secondary mixing reactor 9, suitable for being fed with an organic raw material, preferably sturgeon and/or salmon gonads, and with a secondary solution, comprising sodium chloride and/or citrate, to obtain an intermediate mixture, shredding means 8 suitable for reducing the size of the organic raw material and separator means 10, suitable for separating the intermediate mixture into a de-fatted phase, containing DNA molecules, and a phase containing the fat substances.

. Innovatively, the device which the invention relates to allows faster heat control of the de-fatted mixture, avoiding the deleterious effects of the thermal temperature on the DNA molecules.

. A person skilled in the art may make variations or replace elements with others functionally equivalent to the embodiments of the device and to the method described above so as to satisfy specific requirements.

. For example, the organic raw material may be fed whole to the shredding means 8, being subsequently fed into the secondary mixing reactor 9.

. Such variations also fall within the scope of protection as defined by the following claims.

. In addition, each of the variations described as belonging to one possible embodiment may be made independently of the variations described.

## Claims

1. Method for the preparation of the sodium salt of deoxyribonucleic acid (DNA) comprising the steps of:
- supplying a de-fatted phase containing DNA molecules;
- supplying at least one primary solution, containing sodium chloride, suitable for solubilising or suspending the de-fatted phase;
- feeding the de-fatted phase and the primary solution to a primary mixing reactor (2) so as to obtain a de-fatted mixture; and
- cooling the de-fatted mixture to a pre-defined cooling temperature;
wherein the cooling step comprises a step of taking a portion of the de-fatted mixture from the primary reactor (2), and a step of reintroducing such portion in the primary reactor (2).

2. Method according to claim 1, wherein the step of taking the portion foresees conveying the portion of de-fatted mixture outside the primary reactor (2).

3. Method according to claim 1 or 2, further comprising a step of crushing the DNA molecules contained in the de-fatted mixture, for example through ultrasounds.

4. Method according to claim 3, wherein the cooling and crushing steps occur at least partially simultaneously.

5. Method according to any of the previous claims, wherein the crushing step comprises a step of exposing to ultrasounds at an intensity ranging from 20 to 50 Hz and, preferably, from 30 to 45 Hz.

6. Method according to any of the previous claims, wherein the step of feeding occurs in the absence of fossil meals and/or sodium dodecyl-sulphate.

7. Method according to any of the previous claims, further comprising a step of analysing to determine the crushing degree of the DNA molecules, preferably through a viscosity measurement.

8. Method according to any of the previous claims, wherein the step of supplying the de-fatted phase comprises the steps of:
- supplying an organic raw material, such as sturgeon and/or salmon gonads;
- supplying at least one secondary solution, comprising sodium citrate and/or chloride;
- shredding the organic raw material so as to reduce its size in relation to the starting size;
- mixing the organic raw material and the secondary solution to obtain an intermediate mixture; and
- separating, for example by centrifuging, the intermediate mixture into the de-fatted phase, containing the DNA molecules, and a phase containing the fat substances.

9. Method according to any of the previous claims, further comprising the steps of:
- concentrating the de-fatted mixture, for example by ultra-filtration under pressure, so as to select the DNA molecules having a mass greater than a predefined molecular mass; and
- crystallising, for example from ethanol, the DNA molecules having a mass greater than the predefined molecular mass.

10. Sodium salt obtained through the method according to any of the previous claims.

11. Device (1) for the preparation of the sodium salt of deoxyribonucleic acid (DNA) comprising:
- a primary mixing reactor (2), suitable for being fed with a de-fatted phase containing DNA molecules and with at least one primary solution containing sodium chloride to obtain a de-fatted mixture;
- heat exchange means, operatively connected to the primary reactor (2) to regulate its temperature; **characterised in that** it further comprises circulation means (3, 4), such as a peristaltic pump (3), suitable for taking a portion of the de-fatted mixture from the primary reactor (2), and for reintroducing such portion in the primary reactor (2).

12. Device according to claim 11, wherein the circulation means (3, 4) are suitable for conveying the portion of de-fatted mixture outside the primary reactor (2).

13. Device according to claim 11 or 12, further comprising crushing means (5', 5"), for example a piezoelectric ultrasound generator, operatively connected to the primary reactor (2) so as to crush the DNA molecules contained in the de-fatted mixture.

14. Device according to any of the claims from 11 to 13, wherein the primary reactor (2) defines a heat exchange cavity (6) suitable for the passage of a heat exchange liquid, suitable for being heated or cooled, and wherein the heat exchange means comprise said cavity (6).

15. Device according to claim 13 or 14, wherein the heat exchange means comprise at least one heat exchanger (7', 7"), through which the de-fatted mixture and a heat exchange liquid pass in reciprocal thermal contact, and wherein the crushing means (5', 5") and the heat exchanger (7', 7") are integrated in a single assembly.

16. Device according to any of the claims from 11 to 15, further comprising:
- a secondary mixing reactor (9), suitable for being fed with an organic raw material, such as sturgeon and/or salmon gonads, and with a secondary solution, comprising sodium citrate and/or chloride, to obtain an intermediate mixture;
- shredding means (8) suitable for reducing the size of the organic raw material;
- separator means (10), suitable for separating the intermediate mixture into the de-fatted phase, containing the DNA molecules, and a phase containing the fat substances;
- concentrator means, for example filters for ultra-filtration under pressure, suitable for concentrating the de-fatted mixture so as to select the DNA molecules having a mass greater than a predefined molecular mass; and
- crystallisation means, for example from ethanol, suitable for crystallising the DNA molecules having a mass greater than the predefined molecular mass.
